# EUROPÄISCHE PATENTANMELDUNG

(11) **EP 0 598 938 A1**
(43) Veröffentlichungstag der Anmeldung: **01.06.1994**
(21) Anmeldenummer: 92120037.4
(22) Anmeldetag: 25.11.1992
(51) Int. Cl.: A61N 2/06

(54) **Vorrichtung zum Anbringen von Dauermagneten am menschlichen Körper**

(71) Anmelder: FIRMA FRANZ BREUNING, D-75172 Pforzheim (DE)
(72) Erfinder: Breuning, Marcus, W-7530 Pforzheim (DE); Hieber, Fritz E. W., Dr. med., W-7570 Baden-Baden (DE)
(74) Vertreter: Trappenberg, Hans

(57) **Zusammenfassung**

Zur unblutigen Anwendung der Ohrakupunktur ist es bekannt, die Akupunktur-Punkte und -Linien durch galvanische Einwirkung zu stimulieren. Es hat sich gezeigt, wohl aufgrund des magnetoelektrischen Effekts, daß auch eine Stimulation mittels magnetischer Kräfte, die allerdings sehr lange einwirken müssen, möglich ist. Diese Dauereinwirkung wird nach der Erfindung bei der Ohrakupunktur dadurch erreicht, daß ästhetisch ansprechender Ohrschmuck mit Dauermagneten bestückt ist.

## Beschreibung

Die Erfindung betrifft eine Vorrichtung zum Anbringen von Dauermagneten am menschlichen Körper und damit zum Einwirken magnetischer Kräfte auf menschliches Gewebe, insbesondere auf das Gewebe der mit Akupunktur-Punkten und -Linien belegten Ohrmuschel.

Obwohl noch nicht streng wissenschaftlich erforscht und belegt, hat sich doch die Akupunktur bei Diagnose und Therapie des menschlichen Körpers etabliert. Bei der von alters her übernommenen Akupunktur werden Nadeln aus verschiedenen Metallen in streng umgrenzte Areale des menschlichen Körpers eingestochen und entfalten dort die jeweils gewünschten Wirkungen. Um Irritationen des Gewebes, insbesondere Infektionen an der Einstichstelle, möglichst weitgehend zu verhindern, dürfen die eingestochenen Nadeln allerdings nur kurzfristig im Körper belassen werden. Tatsächlich entfalten sich die gewünschten Wirkungen, je nach Behandlungsmethode und zu behandelnder Störung, praktisch sofort beim Einstechen, so daß ein längeres Belassen der Nadeln im Körper nicht notwendig ist. Tritt allerdings diese sofortige Wirkung nicht ein, müssen die Nadeln mit der Gefahr insbesondere von Infektionen auch längere Zeit in der Einstichstelle verbleiben. Insbesondere um diesen Nachteil zu vermeiden und insgesamt die Akupunktur einfacher und auf alle Fälle unblutig zu gestalten, wurde auch die Behandlung der Akupunktur-Punkte und -Linien durch Elektroakupunktur bekannt, bei der diese Punkte und Linien durch Einwirkung elektrischen Stromes stimuliert werden. Hierbei wird in aller Regel der menschliche Körper mit einem Pol einer Stromquelle verbunden, während der Akupunkteur den jenseitigen Pol auf einen Akupunktur-Punkt aufsetzt. Hierbei ist es auch möglich, die beiden Pole der Stromquelle über eine längere Zeit auf den Körper einwirken zu lassen.

Die klassische, von Asien übernommene Akupunktur-Methode weist Akupunktur-Punkte und -Linien verteilt auf die gesamte Körperoberfläche nach. Eine spezielle Akupunktur-Methode beschäftigt sich mit den Akupunktur-Punkten, die sich speziell auf der Ohrmuschel befinden, da nach dieser speziellen Akupunktur-Lehre sich dort alle den jeweiligen Organen des Körpers zuzuschreibenden Akupunktur-Punkte befinden. Auch diese sogenannte Ohr-Akupunktur arbeitet sowohl invasiv, also mit in die Ohrmuschel eingestochenen Nadeln, wie auch mit Einwirkung des elektrischen Stromes.

Es mag mit dem bekannten magnetoelektrischen Effekt zusammenhängen, nach dem ein Magnetfeld eine zur Feldstärke eines elektrischen Feldes proportionale elektrische Polarisation hervorruft, daß magnetische Einwirkungen auf die Akupunktur-Punkte zu gleichen Ergebnissen führen wie eine Stimulierung dieser Punkte durch elektrischen Strom. Auf jeden Fall lehrt die Praxis, daß solche Einwirkungen tatsächlich zu verzeichnen sind, in aller Regel allerdings nicht stimulierend, sondern eher sedierend. Bei vielen Anwendungen der Akupunktur ist jedoch gerade diese sedierende Wirkung sehr erwünscht. Die Einwirkung magnetischer Kräfte auf solche Akupunktur-Punkte folgt jedoch anderen Gesetzen beziehungsweise erfordert andere Voraussetzungen als die Elektroakupunktur. Bei der Elektroakupunktur muß eine galvanische Verbindung mit dem Gewebe des Akupunktur-Punktes hergestellt werden, so daß ein Strom fließen kann. Die magnetischen Kraftlinien hingegen sind unabhängig von der Leitfähigkeit des Gewebes und treten auch dann in das Gewebe ein, wenn die magnetischen Pole durch dazwischengeschobene Materialien oder durch einen Luftspalt vom Gewebe getrennt sind. Allerdings muß dafür gesorgt werden, daß diese magnetischen Kräfte, da deren Einwirkungen doch sehr gering sind, über einen längeren Zeitraum auf das Gewebe einwirken.

Es ist Aufgabe der Erfindung, eine Vorrichtung anzugeben, wie diese längere Einwirkung beziehungsweise Dauereinwirkung auf bestimmte, am Ohr vorhandene Akupunktur-Punkte und -Linien in ästhetisch ansprechender Weise möglich ist. Erreicht wird dies nach der Erfindung durch mit Dauermagneten bestückten Ohrschmuck.

Nicht mehr also wie bei der Akupunktur mit Nadeln oder bei der Elektroakupunktur werden Akupunktur-Punkte und -Linien nur kurzfristig stimuliert, sondern es findet eine Dauereinwirkung dieser Punkte und Linien durch Einwirkungen magnetischer Kräfte statt, da der den Träger beziehungsweise die Trägerin schmückende Ohrschmuck durchaus dauernd, zumindest tagsüber dauernd, getragen werden kann. Da im Ohrschmuck erfindungsgemäß Dauermagnete integriert sind, werden die am Ohr befindlichen Akupunktur-Punkte und -Linien auch unterbrechungsfrei durch die von den Dauermagneten ausgehenden magnetischen Kraftlinien beeinflußt. Damit ist das oben angeführte Ziel einer magnetischen Dauerbeeinflussung der Ohrakupunktur-Punkte beziehungsweise -Linien erreicht.

Nach einer ersten Ausführung der Erfindung kann der Ohrschmuck ein in Form eines offenen Ringes ausgeführtes schmückendes Gebilde sein, das einerseits in die Ohrmuschelgrube einzuhängen ist und sich andererseits an der Ohrmuschelrückseite abstützt. Die einfachste Form eines solchen Ohrschmucks ist eine Kreole. Eine andere Ohrschmuckform mit hohem therapeutischen Wert ergibt sich wiederum durch ein in Form eines offenen Ringes ausgeführten schmückenden Gebildes, das einerseits in die Ohrmuschelgrube einhängbar und andererseits über ein an der Ohrmuschelrückseite zur Ohrmuscheloberseite führenden Verbindungsstück hakenförmig die Ohrmuscheloberseite beim Ansatz an die Kopfhaut (Endhelix) umgreift. Ein ähnliches Schmuckstück mit etwa gleichen therapeutischen Wirkungen ergibt sich dadurch, daß der Ohrschmuck ein in Form eines offenen Ringes ausgeführtes schmückendes Gebilde ist, das hakenförmig die Ohrmuschel oberseitig beim Ansatz an die Kopfhaut (Endhelix) sowie auch mit einem an der Ohrmuschelrückseite zum Ohrläppchen führenden Verbindungsstuck wiederum hakenförmig die Ohrmuschel unterseitig umgreift.

In allen Fällen können die Dauermagnete als Scheibchen oder Plättchen mit einem zum Gewebe weisenden Pol in den Ohrschmuck eingefügt sein oder es können auch Stabmagnete Verwendung finden, bei denen beide Magnetpole auf das Gewebe einwirken. Sind mehrere in den Ohrschmuck eingefügte Dauermagnete vorgesehen, so können sie so angebracht sein, daß sie eine jeweils wechselnde oder auch eine jeweils gleiche Polarität aufweisen. Sehr gute Wirkungen werden auch dadurch erzielt, daß zwei oder mehr Magnete das Gewebe der Ohrmuschel zwischen sich einschließen beziehungsweise das dort befindliche Gewebe durchfluten.

Im allgemeinen kann angenommen werden, daß die beschriebenen Ohrschmuckformen mit den integrierten Dauermagneten auf große Areale der Ohrmuschel und damit auch auf die dort befindlichen Akupunktur-Punkte und -Linien einwirken, so daß der gewünschte stimulierende oder sedierende Effekt mit Sicherheit erreicht wird.

Auf der Zeichnung sind Ausführungsbeispiele des Erfindungsgegenstandes schematisch dargestellt, und zwar zeigen:
- Fig. 1: einen in die Ohrmuschelgrube eingehängten, kreolenförmigen Ohrschmuck,
- Fig. 2: einen ebenfalls in die Ohrmuschelgrube eingehängten, rückseitig die Ohrmuschel umgreifenden Ohrschmuck,
- Fig. 3: einen ober- und unterseitig die Ohrmuschel umgreifenden, rückseitig verbundenen Ohrschmuck,
- Fig. 4: einen Ohrschmuck, der ähnlich wie Fig. 2 in die Ohrmuschelgrube eingehängt ist und oberseitig die Ohrmuschel umgreift,
- Fig. 5: die Ansicht einer Kreole mit eingefügten Magneten,
- Fig. 6: die Ansicht eines umgreifenden Ohrschmucks mit eingefügten Magneten.

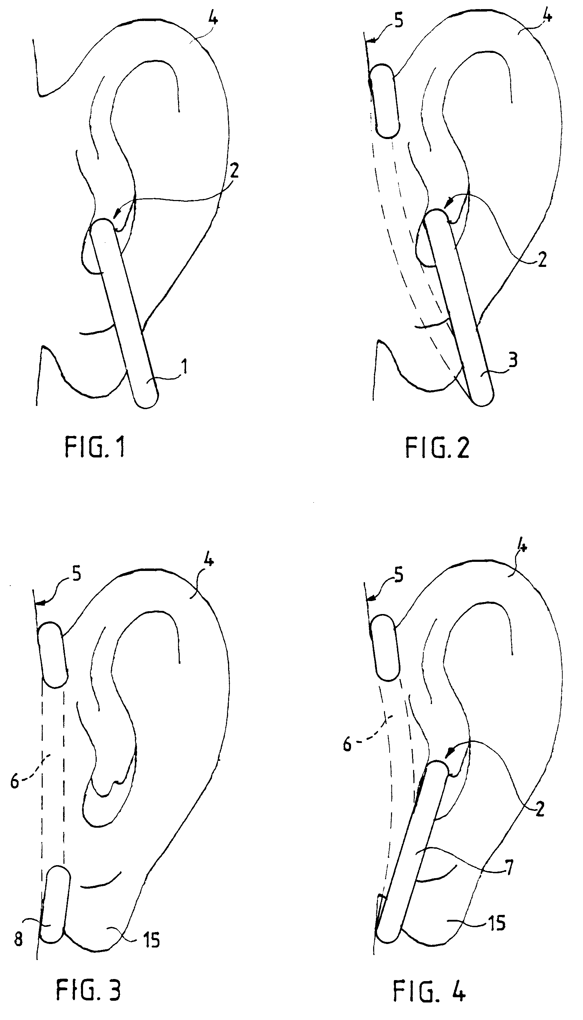

Der kreolenförmige Ohrschmuck (1) nach Fig. 1 wird vorderseitig in die Ohrmuschelgrube (2) eingehängt, umgreift die Ohrmuschel und stützt sich an deren Rückseite ab. Die in Fig. 2 gezeigte Ausführung eines Ohrschmucks (3) wird ebenfalls vorderseitig in die Ohrmuschelgrube (2) eingehängt, führt jedoch nach unterseitiger Umgehung der Ohrmuschel (4) an deren Rückseite nach oben an den Ansatz zwischen Ohrmuschel (4) und Kopfhaut (5) und umgreift dort hakenförmig die Ohrmuschel (4). Gleiches gilt auch für die Ausführung nach Fig. 4, wobei jedoch das auf der Rückseite verlaufende Verbindungsstück (6) dieses Ohrschmucks (7) in der Furche zwischen der Kopfhaut (5) und der Rückseite der Ohrmuschel (4) liegt. Dies gilt auch für die in Fig. 3 gezeigte Ausführung, bei der die Ohrmuschel (4) sowohl oberseitig wie auch unterseitig hakenförmig umgriffen wird. Selbstverständlich können die nach vorne weisenden Schauseiten des Ohrschmucks (1, 3, 7, 8) schmückend ausgestaltet und auch mit Schmucksteinen etc. versehen sein.

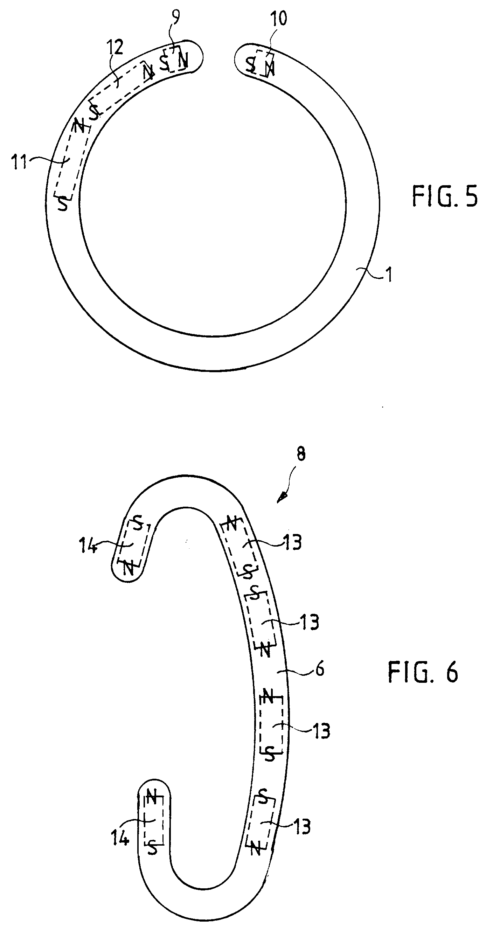

Ein kreolenförmiger, mit Dauermagneten versehener Ohrschmuck in Form einer Kreole ist in Fig. 5 gezeigt. Hierbei sind in die Kreole (1) scheibenförmige Magneten (9, 10) sowie auch Stabmagnete eingefügt, wobei die gegenüberliegenden Pole der Magnete gleichnamig oder auch ungleichnamig sein können. Im gezeigten Beispiel nach Fig. 5 sind diese Pole ungleichnamig, so daß sich durch diese Maßnahme ein starkes Dauermagnetfeld zwischen den freien Stirnseiten der Magnete (10, 11) einstellen wird, das das gesamte zwischen diesen Magnetpolen befindliche Ohrmuschel-Gewebe durchflutet. In Fig. 6 hingegen weisen die einander gegenüberliegenden Stirnflächen der stabförmigen Magnete (13) jeweils die gleiche Polarität auf, so daß sich entlang dieses Verbindungstückes (6) ein starker, auf das Gewebe der Ohrmuschel einwirkender Streufluß ergeben wird. Außerdem ergibt sich ein starkes magnetisches Kraftfeld zwischen den an den hakenförmigen Enden dieses Ohrschmucks eingefügten Magneten (14) und den im Verbindungsstück (6) angebrachten Magneten und damit eine entsprechend starke Durchflutung der dazwischen befindlichen Ohrmuschelareale.

## Patentansprüche

1. Vorrichtung zum Anbringen von Dauermagneten am menschlichen Körper und damit zum Einwirken magnetischer Kräfte auf menschliches Gewebe, insbesondere auf das Gewebe der mit Akupunktur-Punkten und -Linien belegten Ohrmuschel,
gekennzeichnet
durch mit Dauermagneten (9, 10, 11, 12, 13, 14) bestückten Ohrschmuck (1, 3, 7, 8).

2. Vorrichtung nach Anspruch 1,
dadurch gekennzeichnet,
daß der Ohrschmuck (1) ein in Form eines offenen Ringes ausgeführtes schmückendes Gebilde ist, das einerseits in die Ohrmuschelgrube (2) einhängbar ist und sich andererseits an der Ohrmuschelrückseite abstützt.

3. Vorrichtung nach Anspruch 2,
dadurch gekennzeichnet,
daß das schmückende Gebilde eine Kreole (1) ist.

4. Vorrichtung nach Anspruch 1,
dadurch gekennzeichnet,
daß der Ohrschmuck ein in Form eines offenen Ringes ausgeführtes schmückendes Gebilde ist, das einerseits in die Ohrmuschelgrube einhängbar und andererseits über ein an der Ohrmuschelrückseite verlaufendes Verbindungsstück (6) hakenförmig die Ohrmuscheloberseite beim Ansatz an die Kopfhaut (Endhelix) umgreift.

5. Vorrichtung nach Anspruch 1,
dadurch gekennzeichnet,
daß der Ohrschmuck ein in Form eines offenen Ringes ausgeführtes schmückendes Gebilde ist, das hakenförmig die Ohrmuschel oberseitig beim Ansatz an die Kopfhaut (Endhelix) sowie auch mit einem an der Ohrmuschelrückseite zum Ohrläppchen (15) führenden Verbindungsstück wiederum hakenförmig die Ohrmuschel unterseitig umgreift.

6. Vorrichtung nach einem oder mehreren
der vorhergehenden Ansprüche,
dadurch gekennzeichnet,
daß die Dauermagnete (9, 10, 11, 12, 13, 14) als Scheibchen oder Plättchen mit einem zum Gewebe weisenden Pol in den Ohrschmuck (1, 3, 7, 8) eingefügt sind.

7. Vorrichtung nach einem oder mehreren
der vorhergehenden Ansprüche,
dadurch gekennzeichnet,
daß die Dauermagnete (11, 12, 13, 14) als am Gewebe der Ohrmuschel (4) anliegende Stabmagnete in den Ohrschmuck (1, 3, 7, 8) eingefügt sind.

8. Vorrichtung nach einem oder mehreren
der vorhergehenden Ansprüche,
dadurch gekennzeichnet,
daß die Dauermagnete (9, 10, 11, 12, 13, 14) bei mehreren in den Ohrschmuck (1, 3, 7, 8) eingefügten Dauermagneten so angebracht sind, daß sie eine jeweils wechselnde Polarität aufweisen.

9. Vorrichtung nach einem oder mehreren
der vorhergehenden Ansprüche,
dadurch gekennzeichnet,
daß die Dauermagnete (9, 10, 11, 12, 13, 14) bei mehreren in den Ohrschmuck (1, 3, 7, 8) eingefügten Dauermagneten so angebracht sind, daß ihre gegenüberliegenden Stirnseiten jeweils die gleiche Polarität aufweisen.

10. Vorrichtung nach einem oder mehreren
der vorhergehenden Ansprüche,
dadurch gekennzeichnet,
daß die Dauermagnete (9, 10, 11, 12, 13, 14) bei mehreren in den Ohrschmuck (1, 3, 7, 8) eingefügten Dauermagneten so angebracht sind, daß sie bei unterschiedlicher Polarität das zwischen ihnen befindliche Gewebe durchfluten.
